(19) Europäisches Patentamt
European Patent Office
Office européen des brevets

(11) Numéro de publication : **0 298 830 B1**

(12) **FASCICULE DE BREVET EUROPEEN**

(45) Date de publication du fascicule du brevet : **27.11.91 Bulletin 91/48**

(51) Int. Cl.[5] : **C07C 45/00**, C07C 29/36, C07D 307/87, C07D 307/32, C07D 307/60, C07D 311/90, C07C 49/16, C07C 31/40

(21) Numéro de dépôt : **88401630.4**

(22) Date de dépôt : **27.06.88**

(54) **Procédé de perfluoroalkylation d'anhydrides d'acides.**

(30) Priorité : **10.07.87 FR 8710176**

(43) Date de publication de la demande : **11.01.89 Bulletin 89/02**

(45) Mention de la délivrance du brevet : **27.11.91 Bulletin 91/48**

(84) Etats contractants désignés : **AT BE CH DE ES FR GB GR IT LI LU NL SE**

(56) Documents cités :
**EP-A- 0 082 252**
**CH-A- 408 048**
**CHEMICAL ABSTRACTS, vol. 86, no. 19, 9 mai 1977, page 519, résumé no. 139560b, Columbus, Ohio, US; A. SEKIYA et al.: "Reaction of heptafluoro-methylethylzinc iodide with halides and anhydrides of carboxylic acids", & CHEM. LETT. 1977, (1), 81-4**

(73) Titulaire : **RHONE-POULENC CHIMIE**
**25, quai Paul Doumer**
**F-92408 Courbevoie Cédex (FR)**

(72) Inventeur : **Tordeux, Marc**
**1 rue Seignelay**
**F-92330 - Sceaux (FR)**
Inventeur : **Wakselman, Claude**
**5, rue Chanez**
**F-75016 - Paris (FR)**
Inventeur : **Francese, Catherine**
**Résidence des Coquelicots 11, rue de Chevilly**
**F-92240 - L'Hay/Les/Roses (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

**EP 0 298 830 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de cétones perfluoroalkylées ou d'alcools perfluoroalkylés. Elle concerne plus précisement un procédé de perfluoroalkylation d'anhydrides d'acides.

Il est connu par exemple selon l'article de Donald SHAW et de Terrence C. TUOMINEN paru dans Synthetic Communications 15 (14), 1291-1297 (1985) de préparer l'hydroxy-3 trifluorométhyl-3 phtalide, utile comme l'agent régulateur de la croissance des plantes, en trois étapes. Les matières premières utilisées sont le bromotoluène et un amide de l'acide trifluoracétique de formule $CF_3$ $CONCH_3$ $(OCH_3)$ en présence de magnésium dans l'éther. Les matières premières, et particulièrement l'amide de l'acide trifluoracétique, ne sont pas couramment disponibles. En plus, l'utilisation d'un magnésien fortement réactif a toujours fait reculer l'industrie d'un point de vue économie de procédé et sécurité.

Il est encore connu d'après un article de PRABHU EAPEN, et TAMBORSKI paru dans J. Org Chem 1984, 49, 2792-2795 de préparer le même produit que dans l'article précédent, par un procédé dibromé que l'on condense sur le butyllithium à −110°C. L'utilisation d'alkyllithien a toujours été évitée dans l'industrie pour des normes de sécurité car ces dérivées sont très inflamables.

De même, il est encore connu selon le brevet CH-A-408048 (Unilever) de Jan Boldingh et Guido Lardelli de faire une alkylation directe d'anhydrides d'acides par des dérivés organométalliques.

Toutefois, le brevet EP-A-0082252 de Nobuo Ishikawa et Tomoya Kitazume signale l'impossibilité de réaliser une perhalogénoalkylation d'anhydrides d'acides par l'intermédiaire de dérivés perhalogénoalkymagnésiens car ils présentent une trop grande instabilité du fait de l'élimination de fluorure de magnésium.

Ce même brevet décrit une perfluoroalkylation de composés aromatiques plus particulièrement des cétones et des aldéhydes passant par une étape de formation d'organométallique, fluorozinciques et stanniques.

Ces mêmes auteurs, dans un article dans Chemistry Letters pp 1679-1680, 1981 1.19 et 20, décrivent cette même technique, rendue limité du fait de l'emploi indispensable du traitement par ultrasons.

Il est encore connu d'après l'article du Chemistry Letters pp 81-84, 1977 de Nobuo Ishikawa de préparer des cétones fluorées. Selon ce document, une première étape pour la formation de l'iodure de perfluoroalkylzincest nécessaire. Cette réaction se réalise dans des conditions très spécifiques puisque ne fonctionnant que pour des formes ramifiées d'iodure de perfluoroalkylzinc.

Aussi, l'industrie est toujours à la recherche d'un procédé économiquement satisfaisant et sûr d'un point de vue sécurité pour préparer les diverses cétones ou alcools perfluorés demandés par l'industrie agrochimique ou pharmaceutique.

La présente invention a permis d'atteindre cet objectif recherché depuis longtemps par l'industrie : disposer d'un procédé sûr, peu dangereux et économique pour préparer les cétones et alcools perfluorés.

La présente invention concerne un procédé de préparation de cétones et alcools perfluorés par perfluoroalkylation d'anhydrides d'acides par mise en contact d'un anhydride d'acide avec un métal choisi parmi le zinc et le cadmium et un iodure ou un bromure de perfluoroalkyle que l'on rajoute en dernier.

On utilisera de préférence un bromure de perfluoroalkyle.

On entend au nom de la présente invention par anhydride d'acide trois classes de produits :

— les anhydrides d'acides aliphatiques linéaires ou ramifiés
— les anhydrides de diacides
— les anhydrides d'acides aromatiques.

Parmi les anhydrides d'acides aliphatiques linéaires ou ramifiés, on préfère utiliser les anhydrides d'acides contenant 1 à 8 atomes de carbone éventuellement substitués par un groupe alkoxy contenant 1 à 2 atomes de carbone, par un groupe phenoxy, un groupe aralkyl ou un groupe cycloalkyl dont la chaîne alkyl contient 3 à 6 atomes de carbone. On préfère utiliser encore plus avantageusement les anhydrides d'acides aliphatiques linéaires contenant 1 à 4 atomes de carbone et tout particulièrement l'anhydride acétique.

Parmi cette première classe d'acides, on peut citer non limitativement :

- les anhydrides d'acides $\alpha$ et $\beta$ méthoxy ou éthoxybutyriques
- les anhydrides d'acides méthoxy ou éthoxypropioniques
- les anhydrides d'acides méthoxy et éthoxyacétiques
- l'anhydride d'acide phénoxybutyrique
- l'anhydride d'acide phénoxypropionique
- l'anhydride d'acide phénoxyacétique
- l'anhydride d'acide isobutyrique
- l'anhydride d'acide cyclohexylbutyrique
- l'anhydride d'acide cyclohexylpropionique
- l'anhydride d'acide cyclohexylacétique

- l'anhydride d'acide cyclohexylcarboxylique
- les anhydrides d'acide cyclopentylbutyrique, propionique, acétique, carboxylique
- les anhydrides d'acides cyclopropylbutyrique, propionique, acétique, carboxylique
- l'anhydride d'acide phénylacétique
- les anhydrides d'acides phénylbutyrique, propionique.

Parmi les anhydrides de diacides, on peut utiliser les anhydrides de diacides saturés ou insaturés mono ou polycycliques. On préfère utiliser l'anhydride succinique ou l'anhydride phtalique.

Parmi cette deuxième classe d'acides, on peut citer non limitativement :

- l'anhydride succinique
- l'anhydride glutarique
- l'anhydride de l'acide cyclohexanedicarboxylique
- l'anhydride de l'acide norbornène – 5 dicarboxylique – 2, 3
- l'anhydride d'acide maléique
- l'anhydride d'acide citraconique
- l'anhydride d'acide phtalique
- l'anhydride d'acide tétrahydro – 1, 2, 3, 6 phtalique.

Parmi les anhydrides d'acides aromatiques, on peut utiliser les anhydrides d'acides mono ou polycycliques éventuellement substitués par un atome de fluor, de chlore, un groupe alkoxy contenant 1 à 2 atomes de carbone, un groupe trifluorométhyle.

Parmi cette troisième classe d'acides, on peut citer non limitativement :

- l'anhydride benzoïque
- l'anhydride d'acide anisique
- l'anhydride d'acide naphtalénedicarboxylique
- l'anhydride d'acide fluorobenzoïque
- l'anhydride d'acide toluique

Les iodures ou bromures de perfluoroalkyle sont choisis de préférence parmi le bromure de trifluorométhyle et les iodures de perfluoroalkyle dont la chaîne alkyle perfluorée contient 2 à 12 atomes de carbone. Cette préférence n'est pas due à une différence de réactivité des composés bromés ou iodés mais uniquement à une différence de coût. En effet, le bromotrifluorométhane est beaucoup moins cher que l'iodotrifluorométhane et inversement les iodures de perfluoroéthyle, perfluorobutyle.... sont beaucoup moins chers que leurs analogues bromés.

Pour une mise en oeuvre plus aisée de l'invention, on utilise un solvant aprotique polaire et/ou une pyridine;

Les solvants aprotiques polaires sont choisis parmi :

— le diméthylformamide
— le diméthylacétamide
— la N méthylpyrolidone

Les pyridines utilisées sont substituées ou non. On préfère employer la pyridine et la méthylpyridine.

Parmi l'ensemble de ces solvants, on préfère utiliser le diméthylformamide et/ou les pyridines.

Selon une mise en oeuvre avantageuse de l'invention, on utilise de préférence un rapport (atome/mole) du métal, zinc ou cadmium à l'anhydride supérieur ou égal à 1 et inférieur ou égal à 2 et un rapport molaire de l'iodure ou du bromure de perfluoroalkyle à l'anhydride supérieur ou égal à 1. Si l'halogénure de perfluoroalkyle est mis en excès et que l'halogénure utilisé est le bromure de trifluorométhyle, celui-ci sera aisément recyclé car il se présente sous forme gazeuse.

Pour une meilleure mise en oeuvre de la réaction, on préfère maintenir une température inférieure à 115°C et encore plus préférentiellement une température comprise entre 20 et 70°C.

La pression est de préférence supérieure à la pression atmosphérique lorsque l'halogénure de perfluoroalkyle est gazeux et notamment comprise entre 1 et 15 bars.

On opère de préférence en l'absence d'oxygène

Parmi les produits obtenus par le procédé de la présente invention, on peut citer non limitativement :

- la trifluoro – 1,1,1 acétone
- l'hexafluoro – 1,1,1, 3,3,3 methyl – 2 propanol – 2
- la trifluoro 1,1,1 pentanone – 2
- l'hydroxy – 3 trifluorométhyle – 3 isobenzofuranone – 1
- la bis (trifluorométhyle) – 5,5 tétrahydrofuranone – 2
- l'hydroxy – 5 trifluorométhyle – 5 tétrahydrofuranone – 2

Les produits obtenus par le procédé selon l'invention sont notamment utilisés comme agents actifs vis-à-vis de la régulation de la croissance des plantes, ou comme fluides pour les transformateurs (US 3236894).

L'invention va être plus complètement décrite à l'aide des exemples suivants quine doivent pas être considérés comme limitatifs de l'invention.

EXEMPLE 1 : ANHYDRIDE ACETIQUE—Zn

Dans un flacon en verre épais, on met 25 ml de diméthylformamide, 5 ml d'anhydride acétique (0,053 mole) et 5 g de zinc en poudre (0,077 mole).

Le flacon est placé dans un appareil de Parr. On y fait le vide puis on introduit du bromotrifluorométhane jusqu'à une pression de 3,6 bars. On agite le flacon pendant le temps de la réaction, la pression étant maintenue entre 4 et 2,5 bars.

La trifluoro – 1,1,1 acétone est identifiée par RMN du $^{19}F$ et le rendement est déterminé après addition de trifluoro – 2,2,2 éthanol dans le brut de manipulation. Il s'élève à 26%.

RMN$^{19}$F ($CFCl_3$ ext.) = – 79 ppm (s, $CF_3$)

EXEMPLE 2 : ANHYDRIDE ACETIQUE—Cd

Dans un flacon en verre épais, on met 25 ml de pyridine, 10 ml d'anhydride acétique (0,11 mole) et 12 g de cadmium en poudre (0,11 mole).

Le flacon est placé dans un appareil de Parr. On y fait le vide puis on introduit du bromotrifluorométhane jusqu'à une pression de 3,6 bars. On agite le flacon pendant le temps de la réaction, la pression étant maintenue entre 4 et 2,5 bars.

L'hexafluoro – 1,1,1,3,3,3 méthyl – 2 propanol – 2 est identifié par RMN$^{19}$F.

RMN$^{19}$F ($CFCl_3$ ext) = – 80,3 ppm (q, $CF_3$)

EXEMPLE 3 : ANHYDRIDE BUTYRIQUE—Zn

Dans un flacon en verre épais, on met 10 ml d'anhydride butyrique (0,061 mole), 25 ml de pyridine et 6 g de zinc en poudre (0,092 mole).

Le flacon est placé dans un appareil de Parr. On y fait le vide puis on introduit du bromotrifluorométhane jusqu'à une pression de 3,6 bars. Le flacon est agité pendant tout le temps de la réaction ; la pression étant maintenue entre 4 et 2,5 bars.

On filtre puis on hydrolyse avec 30 ml d'acide chlorhydrique à 10% glacé en agitant 30 minutes.

Après extraction à l'éther, lavage à l'eau, séchage sur sulfate de magnésium et évaporation du solvant, la trifluoro – 1,1,1 pentanone – 2 est distillée : Eb = 70-74°C.

Le rendement s'élève à 20%.

| | |
|---|---|
| RMN$^{19}$F ($CFCl_3$ ext.) = | – 76 ppm (s, $CF_3$) |
| RMN$^{1}$H (TMS int.) = | 2,3 ppm (q, $CH_2$) |
| | 1,7 ppm (sext., $CH_2$) |
| | 1 ppm (t, $CH_3$) |

Exemple 4 : Anhydride phtalique—Zn—$CF_3$Br

Dans un flacon en verre épais, on met 5 g d'anhydride phtalique (0,034 mole), 40 ml de pyridine et 4 g de zinc en poudre (0,062 mole).

Le flacon est placé dans un appareil de Parr. On y fait le vide puis on introduit du bromotrifluorométhane jusqu'à une pression de 3,6 bars ; on agite le flacon pendant tout le temps de la réaction, la pression étant maintenue entre 4 et 2,5 bars.

On filtre puis on hydrolyse avec 50 ml d'acide chlorhydrique à 10% glacé en agitant 30 minutes.

Après extraction au chloroforme, lavage à l'eau, séchage sur sulfate de magnésium et évaporation du solvant, l'hydroxy – 3 trifluorométhyle – 3 (3H) isobenzofuranone – 1 est distillée sous pression réduite : Eb = 80-90°C/7,6$10^{-3}$mmHg.

On obtient 4,5 g (61%) PF = 98,2°C (P.F. littérature = 98-100°C)

| | |
|---|---|
| RMN$^{19}$F ($CFCl_3$ ext.) = | – 82 ppm (s, $CF_3$) |
| RMN$^{1}$H (TMS int.) = | 8-7,5 ppm (m, Ar) |
| | 4,4 ppm (OH). |
| RMN$^{13}$C (TMS int.) = | 167,05 ppm (C = 0) |
| | 141,6-135,5-132,4-126,7-126,07-124,03 ppm (C - Ar) |

| | |
|---|---|
| | 117,8 ppm (q, $CF_3$, J = 280 Hz) |
| | 100,3 ppm (q, C-$CF_3$, J = 35 Hz) |
| IR (KBr) = | 3350 $cm^{-1}$ (OH) |
| | 1765 $cm^{-1}$ (C = 0) |
| | 1605 $cm^{-1}$ (C = C-Ar) |
| Masse m/e = | 218 ($M^+$), 201 ($M^+$ – OH), 149 ($M^+$ – $CF_3$) |

EXEMPLE 5 : ANHYDRIDE PHTALIQUE —Cd —$CF_3$ Br

Dans un flacon en verre épais, on met 5 g d'anhydride phtalique (0,0338 mole), 40 ml de pyridine et 5 g de cadmium en poudre (0,0446 mole).

On procède ensuite comme à l'exemple (4).

On obtient 3,7 g d'hydroxy – 3 trifluorométhyle – 3 (3H) isobenzofuranone – 1 (50%).

EXEMPLE 6 : ANHYDRIDE PHTALIQUE —Zn —$C_6F_{13}I$

Dans un ballon on met 20 ml de pyridine, 3 g d'anhydride phtalique (0,021 mole) et 2 g de zinc en poudre (0,031 mole).

On purge à l'Argon et on additione ensuite 10 g de iodo – 1 perfluoro n-hexane (0,0224 mole) en agitant.

On filtre puis on hydrolyse avec 20 ml d'acide chlorhydrique à 10% glacé en agitant 30 minutes.

Après extraction au chloroforme, lavage à l'eau, séchage sur sulfate de magnésium et évaporation du solvant, l'hydroxy – 3 (tridécafluoro n-hexyl) – 3 (3H) isobenzofuranone – 1 est distillée sous pression réduite : Eb = 94°C / $410^{-2}$ mmHg.

On obtient 2 g (21%) de produit qui cristallisent :

PF = 107,3°C.

RMN$^{19}$F ($CFCL_3$ ext.) = – 81,3 ppm (E, $CF_3$) ; – 118,7 à – 128,3 ppm (m, 10 F).

RMN$^1$H (TMS int.) = 8-7,5 ppm (m, 4H Ar) ; 4,3 ppm (OM).

EXEMPLE 7 : ANHYDRIDE SUCCINIQUE

Dans un flacon en verre épais, on met 5 g d'anhydride succinique (0,05 mole), 50 ml de pyridine et 4 g de zinc en poudre (0,0615 mole).

Le flacon est placé dans un appareil de Parr. On y fait le vide puis on introduit du bromotrifluorométhane jusqu'à une pression de 3,6 bars, on agite le flacon pendant toute la durée de la réaction ; la pression étant maintenue entre 4 et 2,5 bars.

On filtre puis on hydrolyse avec 50 ml d'acide chlorhydrique à 10% glacé en agitant 30 minutes.

Après extraction à l'éther, lavage à l'eau, séchage sur sulfate de magnésium et évaporation du solvant, les deux produits obtenus sont séparés par chromatographie en phase gazeuse (colonne 30% SE 30 sur chromosorb PAW 45/60 mesh) à 140°C.

Il s'agit de la bis(trifluorométhyle) – 5,5 tétrahydrofuranone – 2 et de l'hydroxy – 5 trifluorométhyle – 5 tétrahydrofuranone – 2 avec des rendements respectifs de 40% et 18%.

• di(trifluorométhyle) 5,5 tétrahydrofuranone 2

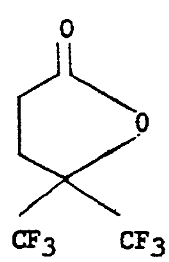

| | |
|---|---|
| RMN$^{19}$F ($CFCl_3$ ext.) : | – 77,3 ppm (s, $CF_3$) |
| RMN$^1$H (TMS int.) : | 3-2,4 ppm (m, $CH_2$ – $CH_2$) |
| RMN$^{13}$C (TMS int.) : | 172,8 ppm (C = 0) |
| | 122,2 ppm (q, $CF_3$, J = 285 Hz) |

$CF_3$
*80,8 ppm (sept, C, J = 32 Hz)*
$CF_3$
26,2 ppm et 23,2 ppm ($CH_2$, $CH_2$)

• Hydroxy – 5 trifluorométhyle – 5 tétrafuranone 2

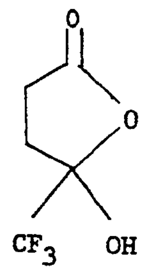

| | |
|---|---|
| RMN[19]F ($CFCl_3$ ext.) : | – 84,7 ppm (s, $CF_3$) |
| RMN[1]H (TMS int.) : | 6 ppm (OH) |
| | 3,2-2,1 ppm (m, $CH_2 - CH_2$) |
| RMN[13]C (TMS int.) : | 176,04 ppm (C = 0) |
| | 122 ppm (q, $CF_3$, J = 287 Hz) |
| | 102,3 ppm (q, C – $CF_3$, J = 35 Hz) |
| | 28,5 ppm et 27,8 ppm ($CH_2 - CH_2$) |

| Ex | Anhydride de départ | Conditions opératoires | Produits obtenus | Rdt |
|---|---|---|---|---|
| 8 | Anhydride benzoïque<br>$(PhCO)_2O$ | 15g (0,064 mole)<br>25 ml pyridine<br>ou DMF<br>5g Zn (0,077 mole)<br>20°C | 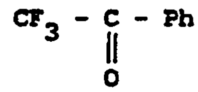<br>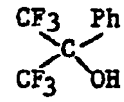 | 5 %<br>10 % |
| 9 | Anhydride naphtalique<br>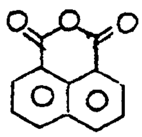 | 5g (0,025 mole)<br>50 ml pyridine<br>3g Zn(0,046 mole)<br>20°C | 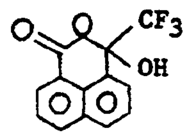 | 10 % |
| 10 | Anhydride maléique<br>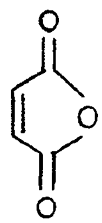 | 10g (0,102 mole)<br>25 ml pyridine<br>ou DMF<br>7g Zn (0,108 mole)<br>20°C | 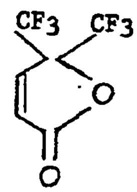 | 5 % |
| 11 | Anhydride citraconique<br>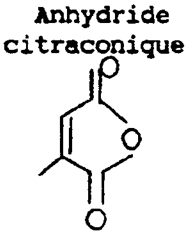 | 5g (0,045 mole)<br>25 ml pyridine<br>3,5g Zn(0,054 mole)<br>20°C | 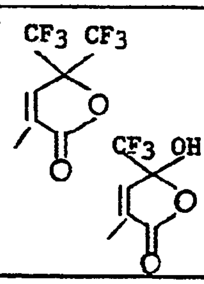 | 5 %<br>5 % |

## Revendications

1. Procédé de préparation de cétones et alcools perfluorés par perfluoroalkylation d'anhydrides d'acides caractérisé en ce que l'on met en contact un anhydride d'acide avec un métal choisi parmi le zinc et le cadmium, et avec un iodure ou un bromure de perfluoroalkyle que l'on rajoute en dernier.

2. Procédé selon la revendication 1 caractérisé en ce que l'iodure ou le bromure de perfluoroalkyle répond à la formule :

$$X(CF_2)_nF$$

dans laquelle

n est compris entre 1 et 12

et X représente Br quand n = 1

et X représente I quand n est compris entre 2 et 12.

3 Procédé selon la revendication 1 caractérisé en ce qu'on utilise de préférence un bromure de perfluoroalkyle.

4. Procédé selon les revendications de 1 à 3 caractérisé en ce que l'anhydride d'acide est choisi parmi les anhydrides d'acides linéaires ou ramifiés contenant 1 à 8 atomes de carbone, les anhydrides de diacides et les anhydrides d'acides aromatiques.

5. Procédé selon la revendication 4 caractérisé en ce que les anhydrides d'acides linéaires ou ramifiés sont éventuellement substitués par un groupe alkoxy contenant 1 à 2 atomes de carbone, par un groupe phenoxy, un groupe cycloalkyl contenant 3 à 6 atomes de carbone.

6. Procédé selon la revendication 4 caractérisé en ce que les anhydrides d'acides linéaires contiennent de préférence 1 à 4 atomes de carbone.

7. Procédé selon la revendication 4 caractérisé en ce que les anhydrides de diacides sont choisis parmi les anhydrides d'acides saturés succinique, glutarique, cyclohexanedicarboxylique et insaturés, maléique, citraconique, phtalique.

8. Procédé selon la revendication 4 caractérisé en ce que les anhydrides d'acides aromatiques sont choisis parmi les acides benzoïques, naphtaléniques éventuellement substitués par un groupe fluor, chlore, alkoxy contenant 1 à 2 atomes de carbone, alkyle contenant 1 à 2 atomes de carbone, trifluorométhyle.

9. Procédé selon les revendications 1 à 3 caractérisé en ce que les anhydrides d'acides sont choisis parmi l'anhydride acétique, l'anhydride phtalique, succinique.

10. Procédé selon les revendications 1 à 9 caractérisé en ce que l'on utilise un solvant choisi parmi les pyridines et les solvants aprotiques polaires.

11. Procédé selon la revendication 10 caractérisé en ce que le solvant est choisi parmi le diméthylformamide, le diméthylacétamide, là N-méthylpyrolidone.

12. Procédé selon les revendications 1 à 11 caractérisé en ce que le rapport du nombre d'atome-gramme du métal au nombre de moles d'iodure ou de bromure de perfluoroalkyle est compris entre 1 et 2.

13. Procédé selon les revendications 1 à 12 caractérisé en ce que la température de réaction est comprise entre 20 et 70°C.

## Claims

1. A process for the preparation of perfluorinated alcohols and ketones by the perfluoroalkylation of anhydrides of acids characterised by bringing an acid anhydride into contact with a metal selected from zinc and cadmium, and with a perfluoroalkyl iodide or bromide which is added last.

2. A process according to claim 1 characterised in that the perfluoroalkyl iodide or bromide corresponds to the formula :

$$X\,(CF_2)_n\,F$$

wherein :

n is between 1 and 12 and
X represents Br when n = 1 and
X represents I when n is between 2 and 12.

3. A process according to claim 1 characterised by preferably using a perfluoroalkyl bride.

4. A process according to claims 1 to 3 characterised in that the acid anhydride is selected from anhydrides of branched or straight chain acids containing from 1 to 8 carbon atoms, anhydrides of diacids and anhydrides of aromatic acids.

5. A process according to claim 4 characterised in that the anhydrides of branched or straight chain acids are optionally substituted by an alkoxy group containing 1 to 2 carbon atoms, a phenoxy group or a cycloalkyl group containing from 3 to 6 carbon atoms.

6. A process according to claim 4 characterised in that the anhydrides of straight chain acids preferably contain from 1 to 4 carbon atoms.

7. A process according to claim 4 characterised in that the anhydrides of diacids are selected from the anhydrides of saturated succinic, glutaric and cyclohexanedicarboxylic acids and unsaturated maleic, citraconic and phthalic acids.

8. A process according to claim 4 characterised in that the anhydrides of aromatic acids are selected from benzoic and naphthalenic acids optionally substituted by a fluorine group, a chlorine group, an alkoxy group containing 1 to 2 carbon atoms, an alkyl group containing 1 to 2 carbon atoms or a trifluoromethyl group.

9. A process according to claims 1 to 3 characterised in that the acid anhydrides are selected from acetic anhydride, phthalic anhydride and succinic anhydride.

10. A process according to claims 1 to 9 characterised by using a solvent selected from pyridines and aprotic polar solvents.

11. A process according to claim 10 characterised in that the solvent is selected from dimethylformamide, dimethylacetamide and N-methylpyrolidone.

12. A process according to claims 1 to 11 characterised in that the ratio of the number of gram atoms of metal to the number of moles of perfluoroalkyl iodide or bromide is between 1 and 2.

13. A process according to claims 1 to 12 characterised in that the reaction temperature is between 20 and 70°C.

## Patentansprüche

1. Verfahren zur Herstellung von perfluorierten Ketonen und Alkoholen durch Perfluoralkylierung von Säureanhydriden, dadurch gekennzeichnet, daß man ein Säureanhydrid mit einem Metall, ausgewählt aus Zink und Cadmium und mit einem Perfluoralkyliodid oder -bromid, das als letztes zugegeben wird, in Berührung bringt.

2. Verfahren nach Anspruch 1, dadurch gekennzeichnet, daß das Perfluoralkyliodid oder -bromid der Formel

$$X(CF_2)_nF$$

entspricht, in der
n im Bereich von 1 bis 12 liegt und
X Br bedeutet, wenn n = 1 und
X I bedeutet, wenn n im Bereich von 2 bis 12 liegt.

3. Verfahren nach Anspruch 1, dadurch gekennzeichnet, daß man vorzugsweise ein Perfluoralkylbromid verwendet.

4. Verfahren nach den Ansprüchen 1 bis 3, dadurch gekennzeichnet, daß das Säureanhydrid unter den Anhydriden von linearen oder verzweigten Säuren mit 1 bis 8 Kohlenstoffatomen, den Anhydriden von Dicarbonsäuren und den Anhydriden von aromatischen Säuren ausgewählt wird.

5. Verfahren nach Anspruch 4, dadurch gekennzeichnet, daß die Anhydride von linearen oder verzweigten Säuren gegebenenfalls mit einer Alkoxygruppe mit 1 bis 2 Kohlenstoffatomen, mit einer Phenoxygruppe oder mit einer Cycloalkylgruppe mit 3 bis 6 Kohlenstoffatomen substituiert sind.

6. Verfahren nach Anspruch 4, dadurch gekennzeichnet, daß man Anhydride von linearen Säuren, die vorzugsweise 1 bis 4 Kohlenstoffatome enthalten, verwendet.

7. Verfahren nach Anspruch 4, dadurch gekennzeichnet, daß man die Anhydride von Dicarbonsäuren aus den Anhydriden der gesättigten Säuren Bernsteinsäure, Glutarsäure, Cyclohexandicarbonsäure und der ungesättigten Säuren Maleinsäure, Citraconsäure, Phthalsäure auswählt.

8. Verfahren nach Anspruch 4, dadurch gekennzeichnet, daß die Anhydride von aromatischen Säuren ausgewählt werden aus Benzoesäuren und Naphtoesäuren, die gegebenenfalls mit einem Fluor- oder Chloratom oder einer Alkoxygruppe mit 1 bis 2 Kohlenstoffatomen, einer Alkylgruppe mit 1 bis 2 Kohlenstoffatomen oder einer Trifluormethylgruppe substituiert sind.

9. Verfahren nach Anspruch 1 bis 3, dadurch gekennzeichnet, daß die Säureanhydride aus Essigsäureanhydrid, Phthalsäureanhydrid, Bernsteinsäureanhydrid ausgewählt werden.

10. Verfahren nach den Ansprüchen 1 bis 9, dadurch gekennzeichnet, daß man ein Lösungsmittel, ausgewählt aus den Pyridinen und den aprotischen polaren Lösungsmitteln, verwendet.

11. Verfahren nach Anspruch 10, dadurch gekennzeichnet, daß das Lösungsmittel aus Dimethylformamid, Dimethylacetamid und N-Methylpyrrolidon ausgewählt wird.

12. Verfahren nach den Ansprüchen 1 bis 11, dadurch gekennzeichnet, daß das Verhältnis von Anzahl gAtom Metall zur Anzahl Mole Perfluoralkyliodid oder -bromid 1 bis 2 beträgt.

13. Verfahren nach den Ansprüchen 1 bis 12, dadurch gekennzeichnet, daß die Reaktionstemperatur im Bereich von 20 bis 70°C liegt.